# EUROPÄISCHE PATENTSCHRIFT

(11) **EP 0 759 296 B1**
(45) Veröffentlichungstag und Bekanntmachung des Hinweises auf die Patenterteilung: **19.09.2001**
(21) Anmeldenummer: 96111483.2
(22) Anmeldetag: 17.07.1996
(51) Int. Cl.: A61K 31/135, A61K 9/00, A61K 9/20

(54) **Schnell zerfallende Arzneiform von Tramadol oder einem Tramadolsalz**
Rapidly disintegrating formulation comprising tramadol or a tramadol salt
Composition pharmaceutique de tramadol ou d'un sel de tramadol à délitement rapide

(30) Priorität: 19.08.1995 DE 19530575
(43) Veröffentlichungstag der Anmeldung: 26.02.1997
(73) Patentinhaber: Grünenthal GmbH, 52078 Aachen (DE)
(72) Erfinder: Bartholomäus, Johannes Heinrich Antonius, Dr., 52080 Aachen (DE); Betzing, Jürgen, Dr., 52062 Aachen (DE)

(56) Entgegenhaltungen:
- EP-A- 0 124 027
- EP-A- 0 147 780
- DE-A- 4 329 794

## Beschreibung

Die Erfindung betrifft bindemittelfreie Tabletten von Tramadol oder einem Tramadolsalz zur oralen Applikation.

Um bei festen Arzneiformen wie Tabletten eine möglichst rasche, definierte Wirkstofffreisetzung zu erzielen, muß ein sehr schneller Zerfall der Arzneiform im Freisetzungsmedium erreicht werden. Der Zerfall, das heißt das Auseinanderfallen der Tabletten in einzelne Granulatpartikel, wird von zahlreichen Faktoren beeinflußt: So können Bindemittel (Granuliermittel), Gleitmittel, Füllstoffe und bei Füllstoffen insbesondere deren Löslichkeit die Zerfallsgeschwindigkeit stark herabsetzen. Ferner haben Größe und Form der Partikel sowie die Tablettenhärte, die von der bei der Herstellung angewendeten Presskraft abhängt, einen großen Einfluß auf die Zerfallsgeschwindigkeit. In vielen Fällen nimmt die Zerfallzeit ab einer bestimmten Tablettenhärte signifikant zu.

Die Zerfallsgeschwindigkeit von Tabletten läßt sich durch Einsatz von Sprengmitteln erhöhen. Sprengmittel sind Substanzen, die in der Lage sind, das Zerfallen von Tabletten in Kontakt mit Wasser, Pufferlösungen oder Verdauungssäften zu beschleunigen. Beispiele bekannter Sprengmittel sind Stärke, niedrig substituierte Natrium-Carboxymethylcellulosen, niedrig substituierte Hydroxypropylcellulosen, Calciumcarboxymethylcellulosen, Alginsäure, quervernetzte Carboxymethylcellulosen und quervernetzte Polyvinylpyrrolidone.

Darüber hinaus ist bekannt, daß die Wirkung von Sprengmitteln sowie der Zerfall von Tabletten durch leicht wasserlösliche Hilfsstoffe und/oder Wirkstoffe stark beeinflußt werden kann, da durch Volumenverminderung während des Auflösens der Begleitstoffe der Tablettenzerfall behindert wird. Hinzu kommt, daß wasserlösliche Stoffe in gewissem Umfang Bindemitteleigenschaften besitzen und Kapillaren in der Tablette durch schnelle Bildung einer hoch konzentrierten Diffusionsgrenzschicht verschlossen werden. Für Tabletten mit in Wasser schwer löslichen Wirkstoffen wird daher in WO 87/01936 vorgeschlagen, wasserlösliches Mannitol gegebenenfalls zusammen mit einem Sprengmittel aufzuschmelzen und anschließend zu mahlen. Die hierbei entstehenden gröberen Partikel mit einer bevorzugten Korngröße zwischen 0,1 und 0,6 mm besitzen eine schlechtere Wasserlöslichkeit als unbehandeltes Mannitol. Tabletten mit einem in Wasser schwer löslichen Wirkstoff, die mit dem aufgeschmolzenen und gemahlenen Mannitol gepreßt wurden, zerfallen in Wasser sehr schnell.

Aus der europäischen Patentanmeldung EP 124 027 sind ebenfalls schnell auflösende Tabletten für schwer wasserlösliche Wirkstoffe bekannt. Diese enthalten den Wirkstoff in einer bestimmten Korngrößenverteilung in Kombination mit mikrokristalliner Cellulose und Stärke.

Zur Erzielung einer guten Sprengmittelwirkung können gemäß der Auslegeschrift DE 16 17 343 Sprengmittel, Bindemittel und Hilfsstoffe zusammen suspendiert und anschließend sprühgetrocknet werden. Die erhaltene sprühbetrocknete Hilfsstoffmischung ermöglicht einen schnellen Zerfall der Tabletten. Ferner sind aus der europäischen Patentanmeldung EP 130 683 sprühgetrocknete Tabletten bekannt, die als Wirkstoff N-Acetyl-p-aminophenol sowie teilweise gelatinierte Stärke, gegebenenfalls in Kombination mit mikrokristallinen Cellulosen als zusätzlichem Zerfallsmittel enthalten. Die Zerfallszeit dieser sprühgetrockneten Tabletten mit pharmazeutisch annehmbaren Härten ist mit 3 bis 5 Minuten jedoch als mäßig einzustufen.

Aus US 3,181,998 ist bekannt, daß der durch Bindemittel verursachten Verzögerung des Zerfalls von Preßlingen entgegengewirkt werden kann, wenn in die Arzneimittelformulierung Enzyme in trockenem Zustand eingearbeitet werden. Die Enzyme werden bei Kontakt mit Wasser oder Verdauungssäften aktiviert und beschleunigen den Zerfall der Tabletten durch Spaltung der als Bindemittel eingesetzten Stärken, Cellulosenderivate oder Gelatinen.

Ein weiterer Faktor, der die Zerfallsgeschwindigkeit von Tabletten beeinflußt, ist die Tablettenhärte. Hohe Preßkräfte während des Tablettierens bewirken große Tablettenhärten, das heißt hohe Bindungskräfte innerhalb des Preßlings, die den Zerfall bei Kontakt mit wäßrigem Medium erschweren. In US 5,254,355 wird versucht, dieses Problem dadurch zu lösen, daß Trockenmischungen zu Tabletten mit sehr niedrigen Härten unter 35 N gepreßt werden und anschließend die Härte um mindestens 10 N durch Verglasung der Tablettenoberfläche erhöht wird. Hierdurch wird erreicht, daß im Tabletteninneren die Bindungskräfte weiterhin gering sind und ein guter Zerfall möglich wird.

Bei den aus der Patentanmeldung DE 39 09 520 bekannten Brausetabletten wird der schnelle Zerfall der Tablette durch Hilfsstoffe gefördert, die bei Kontakt mit Wasser CO₂ entwickeln.

Tramadol-Hydrochlorid - (1 RS; 2 RS)-2-[(Dimethylamino)methyl]-1-(3-methoxyphenyl)cyclohexanol, Hydrochlorid - ist ein Analgetikum, das bei starken und mittelstarken Schmerzen wirksam ist. Die sehr gute Wasserlöslichkeit dieses Wirkstoffes hat jedoch bisher die erfolgreiche Entwicklung von in Wasser schnell zerfallenden Tabletten verhindert. Wird beispielsweise Tramadol mit einem unlöslichen Hilfsstoff wie Calciumdihydrogenphosphat und einem Supersprengmittel wie Kollidon® CL gemischt und granuliert, benötigen die erhaltenen Tabletten mit einer Härte von 80 N für den vollständigen Zerfall 5 Minuten. Auch durch Erhöhung des Sprengmittelgehaltes kann der Zerfall nicht beschleunigt werden. Eine Zerfallsgeschwindigkeit von 5 Minuten ist jedoch für eine Tablette, die in Wasser schnell zerfallen und den Wirkstoff rasch freisetzen soll, nicht akzeptabel. Daher bestand die der Erfindung zugrundeliegende Aufgabe in der Entwicklung einer Tramadol oder ein Tramadolsalz enthaltenden Tablette, die in Wasser schnell zerfällt und den Wirkstoff rasch freisetzt, so daß eine sofort trinkbare, wirkstoffhaltige Suspension zur Verfügung steht.

Es wurde gefunden, daß die an die zu entwickelnde Arzneiform gestellten Anforderungen von bindemittelfreien Tabletten erfüllt werden, die Tramadol oder ein Tramadolsalz in Kombination mit mikrokristalliner Cellulose in bestimmten Gewichtsverhältnissen enthalten.

Gegenstand der Erfindung sind dementsprechend bindemittelfreie Tabletten zur oralen Applikation, die mikrokristalline Cellulose und Tramadol oder ein Tramadolsalz im Gewichtsverhältnis von wenigstens 2 : 1 enthalten.

Bevorzugt werden bindemittelfreie Tabletten, in denen das Gewichtsverhältnis von mikrokristalliner Cellulose zu Tramadol oder einem Tramadolsalz wenigstens 3 : 1 und insbesondere wenigstens 4 : 1 beträgt.

Die erfindungsgemäßen Tabletten zerfallen in Wasser sehr schnell und setzen Tramadol oder Tramadolsalz rasch frei. So entsteht unmittelbar nachdem die erfindungsgemäße Tablette mit Wasser in Kontakt gekommen ist, eine trinkfähige, wirkstoffhaltige Suspension. Mit den erfindungsgemäßen Tabletten werden vor der Einnahme die positiven Eigenschaften einer festen Arzneiform wie exakte Dosierung, gute Haltbarkeit, hygienische Verpackungsmöglichkeit und Verzicht auf Konservierungsmittel und bei der Einnahme die positiven Eigenschaften einer flüssigen Arzneiform, beispielsweise leichte Schluckbarkeit und rasches Anfluten des Wirkstoffes im Blut ausgenutzt.

Besonders bevorzugt werden erfindungsgemäße Tabletten, die Stärke insbesondere im Gewichtsverhältnis Stärke zu Tramadol oder Tramadolsalz 1 : 1 enthalten. Bei diesen Tabletten ist der Einfluß der Tablettenhärte auf die Zerfallsgeschwindigkeit deutlich reduziert. Wird die Härte von erfindungsgemäßen Tabletten, die keine Stärke enthalten (Beispiel 1), von 80 N auf 100 N erhöht, steigt die Zerfallszeit von 30 Sekunden auf 120 Sekunden. Dagegen steigt die Zerfallszeit einer stärkehaltigen erfindungsgemäßen Tablette (Beispiel 2) bei einer Erhöhung der Härte von 80 N auf 100 N von 30 Sekunden auf 55 Sekunden. Erfindungsgemäße Tabletten, die Stärke enthalten, zeigen darüber hinaus eine beschleunigte Wirkstofffreisetzung.

In erfindungsgemäßen Tabletten beträgt das Gewichtsverhältnis mikrokristalline Cellulose zu Tramadol/Tramadolsalz zu Stärke wenigstens 2 : 1 : 1. Liegt der Gewichtsanteil von mikrokristalliner Cellulose im Verhältnis zu Tramadol/Tramadolsalz und Stärke darunter, nimmt die Zerfallsgeschwindigkeit der Tabletten stark ab. Auch der Austausch von mikrokristalliner Cellulose gegen wasserlösliche Lactose oder wasserunlösliches Calciumhydrogenphosphat bewirkt eine deutliche Abnahme der Zerfallsgeschwindigkeit.

Die erfindungsgemäßen Tabletten enthalten pro Tablette 5 bis 1000 mg, vorzugsweise 10 bis 200 mg Tramadol und/oder ein Tramadolsalz, insbesondere Tramadolhydrochlorid.

Als fakultative Bestandteile können die erfindungsgemäßen Tabletten 0,5 bis 10 Gewichtsprozent wenigstens eines Sprengmittels, beispielsweise quervernetztes Polyvinylpyrrolidon (PVP-CL), quervernetzte Carboxymethylcellulose und/oder Natriumcarboxymethylstärke, und bis zu 20 Gewichtsprozent geschmacksverbessernde Substanzen, wie Süßstoff, z.B. Natriumsaccharin, Natriumcyclamat und/oder Aspartam, sowie Aromen, z.B. Frucht- und/oder Gewürzaromen, enthalten.

Die Herstellung der erfindungsgemäßen Tabletten erfolgt vorzugsweise durch Mischen der Bestandteile mit anschließender Verpressung.

### Beispiele

Die Tablettenhärte wurde mit einem Heberlein Härtebestimmungsgerät (Modell 2E/205) ermittelt.

### Beispiel 1

Zur Herstellung von 200.000 Tabletten wurden

| | |
|---|---|
| 10.000 g | Tramadolhydrochlorid |
| 44.400 g | mikrokristalline Cellulose |
| 2.000 g | Natriumsaccharin |
| 1.000 g | Pfefferminz-Aroma |
| 2.000 g | Anis-Aroma |
| 400 g | hochdisperses Siliziumdioxid |
| 200 g | Magnesiumstearat |

in einem Containermischer gemischt. Anschließend wurde die Mischung durch ein 0,6 mm Sieb gegeben und erneut in einem Containermischer gemischt. Die nachfolgende Tablettierung wurde auf einer Fette P 2.000-Tablettenpresse durchgeführt. Die erhaltenen Tabletten mit einem Durchmesser von 10 mm und einer mittleren Höhe von 3,2 mm hatten ein mittleres Gewicht von 300 mg und eine zwischen 60 und 80 N liegende Härte.

### Beispiel 2

Unter den in Beispiel 1 angegebenen Bedingungen wurden 200.000 Tabletten aus

| | |
|---|---|
| 10.000 g | Tramadolhydrochlorid |
| 48.400 g | mikrokristalliner Cellulose |
| 10.000 g | Maisstärke |
| 2.000 g | Natriumsaccharin |
| 1.000 g | Pfefferminz-Aroma |
| 2.000 g | Anis-Aroma |
| 400 g | hochdispersem Siliziumdioxid |
| 200 g | Magnesiumstearat |

hergestellt. Die erhaltenen Tabletten mit einem Durchmesser von 10 mm und einer mittleren Höhe von 3,9 mm hatten ein mittleres Gewicht von 370 mg und eine zwischen 60 und 80 N liegende Härte.

### Beispiel 3

Unter den in Beispiel 1 angegebenen Bedingungen wurden 200.000 Tabletten aus

| | |
|---|---|
| 10.000 g | Tramadolhydrochlorid |
| 29.400 g | mikrokristalliner Cellulose |
| 10.000 g | Maisstärke |
| 2.000 g | Natriumsaccharin |
| 1.000 g | Pfefferminz-Aroma |
| 2.000 g | Anis-Aroma |
| 400 g | hochdispersem Siliziumdioxid |
| 200 g | Magnesiumstearat |

hergestellt. Die erhaltenen Tabletten mit einem Durchmesser von 10 mm und einer mittleren Höhe von 3,9 mm hatten ein mittleres Gewicht von 275 mg und eine zwischen 60 und 80 N liegende Härte.

### Beispiel 4

Unter den in Beispiel 1 angegebenen Bedingungen wurden 200.000 Tabletten aus

| | |
|---|---|
| 10.000 g | Tramadolhydrochlorid |
| 20.400 g | mikrokristalliner Cellulose |
| 10.000 g | Maisstärke |
| 2.000 g | Natriumsaccharin |
| 1.000 g | Pfefferminz-Aroma |
| 2.000 g | Anis-Aroma |
| 400 g | hochdispersem Siliziumdioxid |
| 200 g | Magnesiumstearat |

hergestellt. Die erhaltenen Tabletten mit einem Durchmesser von 10 mm und einer mittleren Höhe von 3,9 mm hatten ein mittleres Gewicht von 230 mg und eine zwischen 60 und 80 N liegende Härte.

### Beispiel 5

Unter den in Beispiel 1 angegebenen Bedingungen wurden 200.000 Tabletten aus

| | |
|---|---|
| 10.000 g | Tramadolhydrochlorid |
| 20.140 g | mikrokristalliner Cellulose |
| 10.000 g | Maisstärke |
| 2.000 g | Natriumsaccharin |
| 260 g | PVP-CL |
| 1.000 g | Pfefferminz-Aroma |
| 2.000 g | Anis-Aroma |
| 400 g | hochdispersem Siliziumdioxid |
| 200 g | Magnesiumstearat |

hergestellt. Die erhaltenen Tabletten mit einem Durchmesser von 10 mm und einer mittleren Höhe von 3,9 mm hatten ein mittleres Gewicht von 230 mg und eine zwischen 60 und 80 N liegende Härte.

### Beispiel 6 (Vergleich)

Unter den in Beispiel 1 angegebenen Bedingungen wurden 200.000 Tabletten mit der in Beispiel 2 angegebenen Zusammensetzung hergestellt, wobei anstelle mikrokristalliner Cellulose wasserlösliche Lactose eingesetzt wurde.

### Beispiel 7 (Vergleich)

Unter den in Beispiel 1 angegebenen Bedingungen wurden 200.000 Tabletten mit der in Beispiel 2 angegebenen Zusammensetzung hergestellt, wobei anstelle mikrokristalliner Cellulose unlösliches Calciumhydrogenphosphat eingesetzt wurde.

### Bestimmung der Zerfallszeit und Freisetzungszeit tramadolhaltiger Tabletten

Die Freisetzungszeiten wurden nach Ph. Eur./DAB in einer Blattrührer-Apparatur in 600 ml Magensaft mit einem pH-Wert von 1,2 spektralphotometisch bestimmt. Die Temperatur des Freisetzungsmediums betrug 37°C ± 0,5°C und die Rührgeschwindigkeit 75 Upm. Die Zerfallzeiten wurden mit einem Erweka-Zerfallstester (Z T6-1-D) bestimmt.

Die Ergebnisse sind in der nachfolgenden Tabelle zusammengefaßt:

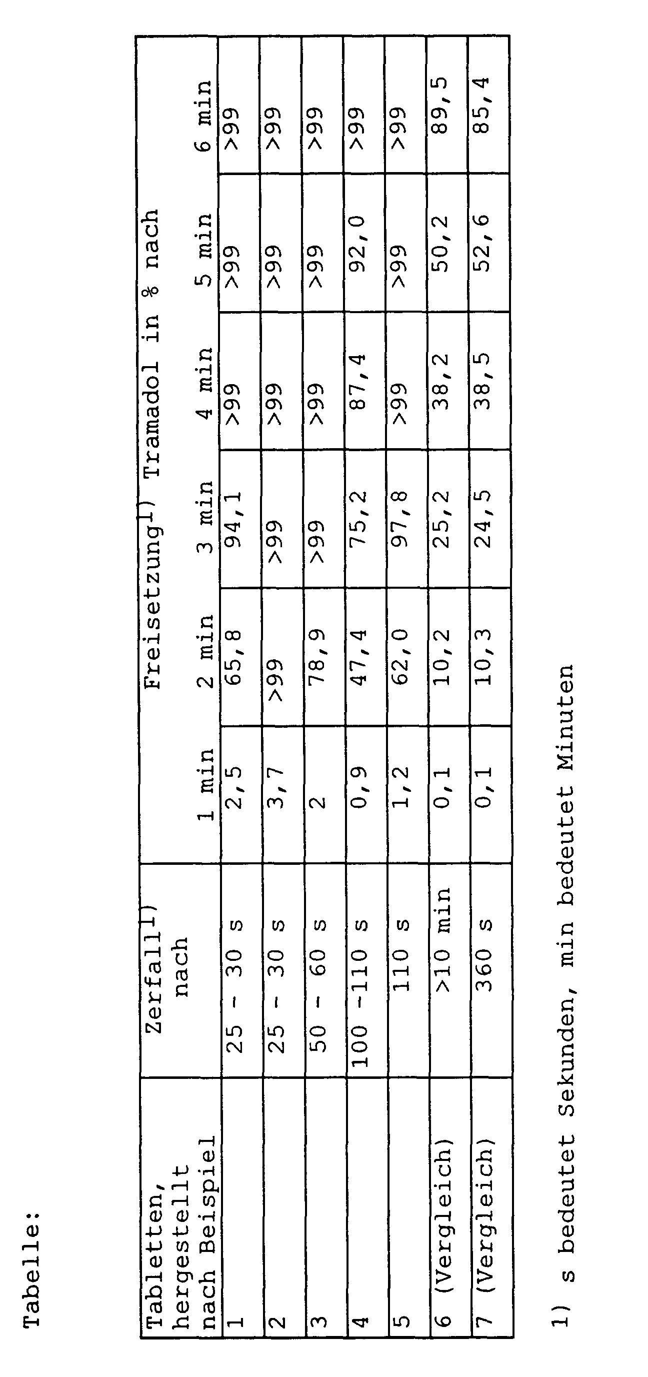

## Patentansprüche

1. Bindemittelfreie Tabletten von Tramadol oder einem Tramadolsalz zur oralen Applikation, enthaltend mikrokristalline Cellulose und Tramadol oder ein Tramadolsalz im Gewichtsverhältnis von wenigstens 2 : 1.

2. Tabletten nach Anspruch 1, **dadurch gekennzeichnet, daß** das Gewichtsverhältnis mikrokristalline Cellulose zu Tramadol oder einem Tramadolsalz wenigstens 3 : 1, vorzugsweise wenigstens 4 : 1 beträgt.

3. Tabletten nach einem oder beiden der Ansprüche 1 bis 2, **dadurch gekennzeichnet, daß** die Tabletten Stärke enthalten.

4. Tabletten nach Anspruch 3, **dadurch gekennzeichnet, daß** das Gewichtsverhältnis Stärke zu Tramadol oder einem Tramadolsalz 1 : 1 beträgt.

5. Tabletten nach einem oder mehreren der Ansprüche 1 bis 4, **dadurch gekennzeichnet, daß** die Tabletten 0,5 bis 10 Gewichtsprozent wenigstens eines Sprengmittels als fakulativen Bestandteil enthalten.

## Claims

1. Binder-free tablets of tramadol or a tramadol salt for oral application, containing micro-crystalline cellulose and tramadol or a tramadol salt in a ratio by weight of at least 2 : 1.

2. Tablets according to claim 1, **characterised in that** the ratio by weight of microcrystalline cellulose to tramadol or a tramadol salt is at least 3 : 1, preferably at least 4 : 1.

3. Tablets according to one or both of claims 1 to 2, **characterised in that** the tablets contain starch.

4. Tablets according to claim 3, **characterised in that** the ratio by weight of starch to tramadol or a tramadol salt is 1 : 1.

5. Tablets according to one or more of claims 1 to 4, **characterised in that** the tablets contain 0.5 to 10 percent by weight of at least one disintegrating agent as an optional constituent.

## Revendications

1. Comprimés de tramadol ou d'un sel de tramadol sans liant, destinés à l'administration orale, contenant de la cellulose microcristalline et du tramadol ou un sel de tramadol dans un rapport en poids d'au moins 2:1.

2. Comprimés suivant la revendication 1, **caractérisés en ce que** le rapport en poids de la cellulose microcristalline au tramadol ou à un sel de tramadol s'élève au moins à 3:1, de préférence au moins à 4:1.

3. Comprimés suivant l'une des revendications 1 et 2 ou les deux, **caractérisés en ce qu'**ils contiennent de l'amidon.

4. Comprimés suivant la revendication 3, **caractérisés en ce que** le rapport en poids de l'amidon au tramadol ou à un sel de tramadol s'élève à 1:1.

5. Comprimés suivant l'une ou plusieurs des revendications 1 à 4, **caractérisés en ce qu'**ils contiennent 0,5 à 10 % en poids d'au moins un agent de désintégration comme composant facultatif.
